Europäisches Patentamt

European Patent Office

Office européen des brevets

(19)

(11) Numéro de publication: **0 088 016**

**A1**

(12) **DEMANDE DE BREVET EUROPEEN**

(21) Numéro de dépôt: **83400383.2**

(22) Date de dépôt: **25.02.83**

(51) Int. Cl.³: **A 61 K 31/195**

(30) Priorité: 01.03.82 US 353469
27.12.82 US 452984

(43) Date de publication de la demande:
07.09.83 Bulletin 83/36

(84) Etats contractants désignés:
BE CH DE FR GB IT LI LU NL SE

(71) Demandeur: PHARMUKA LABORATOIRES
35, Quai du Moulin de Cage
F-92231 Gennevilliers(FR)

(72) Inventeur: Le Fur, Gérard Roger
35, rue du Progrès
F-92350 Plessis Robinson(FR)

(74) Mandataire: Leboulenger, Jean et al,
P C U K PRODUITS CHIMIQUES UGINE KUHLMANN
Service Propriéte Industrielle Tour Manhattan
F-92087 Paris La Défense 2 Cedex 21(FR)

(54) Médicament à base de L-méthionine.

(57) Médicament utile pour le traitement de syndromes dégénératifs ou le traitement de maladies associées à des désordres immunitaires caractérisé en ce qu'il contient en tant que principe actif de la L-méthionine.

EP 0 088 016 A1

Médicament   à base de L-méthionine

La présente invention concerne un nouveau médicament utile
pour le traitement de syndromes dégénératifs tels que, par
exemple la maladie de Parkinson et les troubles associés à
la sénescence ainsi que pour le traitement de maladies associées à des désordres immunitaires, contenant en tant qu'agent
actif de la L-méthionine.

La DL-méthionine (acide amino-2 méthylthio-4 butanoïque) est
considérée comme un acide aminé essentiel pour la croissance
des mammifères car elle procure à l'organisme à la fois les
groupes méthyl et le soufre nécessaires à un processus métabolique normal.

La DL-méthionine joue un rôle important dans la nutrition
animale mais on lui a également attribué des effets bénéfiques
pour la prévention et le traitement de troubles hépatiques
consécutifs à des intoxications par l'arsenic, le tétrachlorure
de carbone... ou pour le traitement des cirrhoses. Cependant,
ces emplois thérapeutiques de la DL-méthionine pour le traitement des troubles hépatiques sont maintenant controversés
et l'on considère qu'il n'existe dans ce domaine aucune preuve
de l'utilité thérapeutique de cet acide aminé (U.S. Dispensatory, 27ème édition p. 792 (1973) Lippincott, Philadelphie).

Il a été maintenant découvert qu'un traitement par L-méthionine
(500 mg/kg i.p. deux fois par jour) augmente chez la souris
nude (souche dépourvue de poils) le nombre des récepteurs
dopaminergiques du striatum ainsi que ceux qui ont été récemment décrits dans la lignée lymphocytaire B (J. Pharm.
Pharmacol. _33_ pp. 102-103 (1981)).

Etant donné que la souche de souris nude est dépourvue de
thymus tous les lymphocytes de la rate sont du type B ; ainsi
cette souche de souris à l'avantage de présenter un modèle
pur du type B.

Les résultats rassemblés dans le tableau I montrent les effets d'un traitement chronique de la souris nude par L-méthionine (500 mg/kg i.p. 2 fois par jour pendant 5 jours) sur la liaison de la $[^3H]$ spiperone.

Les essais, c'est-à-dire liaison de la $[^3H]$ spiperone sur membranes de striatum (Eur. J. Pharmacol. $\underline{50}$, 283, 1978) et sur B-lymphocytes intacts (J. Pharm. Pharmacol. $\underline{33}$, 102, 1981) ont été effectués sur le même animal. Les valeurs pour p indiquent que le groupe traité a été significativement différent du groupe témoin avec une probabilité (p) inférieure à 1 % et 0,1 % respectivement.

## TABLEAU I

| | STRIATUM $[^3H]$ SPIPERONE = 0,3 nM (fmoles/mg/protéine) | B-LYMPHOCYTES $[^3H]$ SPIPERONE = 1 nM (fmoles/$10^6$ cellules) |
|---|---|---|
| TEMOINS | $40 \pm 1$ | $17 \pm 3$ |
| L-METHIONINE | $62 \pm 3$ ** | $37 \pm 5$ * |

\* $p < 0.01$
\*\* $p < 0.001$
$n = 10$

Ces augmentations de liaison de $[^3H]$ spiperone (50 à 120 %) ne correspondent pas à un changement de la constante d'affinité ($K_D$) mais à un changement du nombre de sites de liaison ($B_{max}$). Par exemple, dans le striatum après un traitement chronique par L-méthionine aucun changement de la constante d'affinité ne fut détecté ($K_D$ = 0,17 nM et 0,20 nM chez des témoins et les animaux traités respectivement). En revanche une augmentation du nombre de sites de liaison fût constatée $[B_{max}$ respectivement 302 et 491 fmoles/mg/protéine chez les témoins et les animaux traités].

On considère comme maladies dégénératives des maladies où un ou plusieurs récepteurs ou enzymes décroissent avec le temps. C'est le cas en particulier de la sénescence et de la maladie de Parkinson.

Il est connu que le nombre de récepteurs dopaminergiques diminue lors du vieillissement [J. Pharm. Pharmacol. 30, 448, (1978) et Brain Res. 192, 147 (1980)]. Une diminution du nombre des récepteurs dopaminergiques a été aussi constatée chez des Parkinsoniens non traités [Life Sci. 27, 1587 (1980)].

Dès lors qu'un traitement par L-méthionine antagonise la diminution du nombre des récepteurs dopaminergiques, il devrait antagoniser tous les états pathologiques (comme le parkinson) ou non pathologiques (comme la senescence) où une diminution du nombre de ces récepteurs est impliquée.

C'est ainsi que selon la présente invention la L-méthionine, s'est révélée efficace pour le traitement de la maladie de Parkinson et de la sénescence.

De plus la L-méthionine est très peu toxique puisque, par exemple la dose léthale 50 % est chez le rat de 30 mmol (4,5 g/kg) : voir Clin. Pharmacol. Therap. 9, 485, 1968.

Les observations suivantes concernent l'effet constaté chez des malades atteints de parkinsonisme où il a été constaté, selon la présente invention, qu'un traitement par L-méthionine induit une très bonne amélioration de cette maladie dégénérative.

Onze malades, 5 femmes et 6 hommes (âge moyen 69 ans – limites 63 à 83 ans) atteints de parkinsonisme primaire ont été traités à la posologie quotidienne de 5 g de L-méthionine. Le degré d'atteinte clinique pour chaque cas fut coté selon la

méthode de HOEHN et YAHR (Neurology 17, 427, 1967) : 2 malades étaient au stade I, 3 au stade II, 4 au stade III et 2 au stade IV : tous ces malades n'avaient soit jamais été traités préalablement par des médicaments antiparkinsoniens soit reçu de traitement dans les 4 semaines précédant le début de l'étude.

Chez tous ces malades une très bonne amélioration de l'état clinique fut observée après 2 semaines de traitement par L-méthionine. Notamment sur l'akinésie, les tremblements et aussi la thymie : aucun effet secondaire ne fut constaté, la L-méthionine étant bien tolérée. C'est ainsi que plusieurs malades furent traités pendant plus de 6 mois sans aucun effet indésirable. Comme il avait été préalablement observé sur la souche de souris nude le traitement des malades par L-méthionine induit une augmentation du nombre de sites de binding de la $[^3H]$ spiperone dans les lymphocytes B en l'occurence de $7 \pm 6$ fentomoles pour $10^6$ cellules avant traitement à $95 \pm 15$ fentomoles après traitement.

Il a été rapporté que la L-méthionine peut antagoniser l'efficacité de la L-Dopa dans la maladie de Parkinson [J. Amer. Med. Assoc. 230, 305 (1974)]. Cet antagonisme pourrait être expliqué par une interaction pharmacocinétique entre la L-Dopa et la L-méthionine [Transmethylation, Elsevier pp. 59-66 (1979)]. En conséquence chez des malades parkinsoniens le traitement par L-méthionine ne doit pas être associé à un traitement par L-Dopa.

Selon la présente invention il a également été découvert que la L-méthionine possédait des propriétés immunomodulatrices. Toute modification dans l'équilibre du fonctionnement réciproque des cellules du système immunitaire (polynucléaires, monocytes-macrophages, lymphocytes T, lymphocytes B) aboutit à un état pathologique tel qu'immunodéficit et maladies autoimmunes.

Les propriétés immunomodulatrices de la L-méthionine ont été

- 5 -

0088016

démontrées sur le pouvoir mitogène de la concanavalin (Con A) et du lipopolysaccharide d'E.COLI (LPS) sur des lymphocytes T provenant de rate de souris Balc/C et sur des lymphocytes B provenant de rate de souris nude selon la méthode de THORPE et Coll. (J. Immunol. Methods (1974) 5 n° 4 pp. 387-404).

Les résultats présentés dans les tableaux suivants montrent que la L-méthionine potentialise le pouvoir mitogène de la Con A sur les lymphocytes T et celui du LPS sur les lymphocytes B.

Effet de la L-méthionine sur la synthèse d'ADN induite par la Con A dans des lymphocytes de souris Balc/C.

TABLEAU II

| non stimulé | Con A (2 mcg/ml) | Con A (2 mcg/ml) + L-méthionine [M] | | | |
|---|---|---|---|---|---|
| | | $10^{-2}$ | $10^{-3}$ | $10^{-4}$ | $10^{-5}$ |
| 2378* ± 974 | 173 960 ± 18 448 | 282300 ± 22236 | 295904 ± 39296 | 265346 ± 35736 | 232066 ± 30626 |
| Stimulation (% de Con A) | | + 62 | + 70 | + 52 | + 33 |

* DPM de $[^3H]$ Thymidine/$10^6$ cellules.

Effet de la L-méthionine sur la synthèse d'ADN induite par le LPS dans les lymphocytes de souris nude.

TABLEAU III

| non stimulé | LPS (50 mcg/ml) | LPS (50 mcg/ml) + L-méthionine [M] | | | |
|---|---|---|---|---|---|
| | | $10^{-2}$ | $5\times10^{-3}$ | $2.5\times10^{-3}$ | $10^{-3}$ |
| 5008 ± 356 | 31398 ± 351 | 174234 ± 10100 | 118938 ± 5354 | 67996 ± 900 | 50280 ± 1438 |
| Stimulation (% de LPS) | | + 455 | + 279 | + 116 | + 60 |

La L-méthionine peut être préparée à partir de sources naturelles telles que les hydrolysats de caséine ; ou par fermentation ou encore à partir de méthionine racémique. La méthionine racémique peut être synthétisée selon plusieurs procédés. Habituellement la synthèse industrielle se fait à partir de la β-méthyl mercaptopropionaldéhyde (Kirk OTHMER, Encyclopedia of Chemical Technology, 3ème éd. 2 p. 402 : Wiley Interscience et MERCK INDEX, 9ème éd. p. 5840).

L'isolement de la L-méthionine naturelle peut être effectué par extraction au butanol à partir d'hydrolysats de caséine. [Biochem. J., 26, 1270, (1932)]. La L-méthionine peut aussi être obtenue par des procédés de fermentation (brevet français 2 078 171) ou par résolution du racémique en utilisant par exemple un traitement par une acylase de la N-acétyl-DL-méthionine (brevet allemand 2 105 009).

## EMPLOIS THERAPEUTIQUES

La composition, selon la présente invention peut être utilisée par voie orale chez les mammifères, en particulier chez l'homme. La posologie journalière de la L-méthionine, fonction du poids corporel, est chez le malade adulte comprise entre 1 et 20 g par jour de préférence entre 1 et 10 g avec des doses unitaires comprises entre 2,5 g et 500 mg.

## FORME PHARMACEUTIQUE

Puisque la posologie quotidienne est comprise entre 1 et 20 g la L-méthionine peut être présentée sous diverses formes telles que tablettes, capsules, granulés ou suspensions. Jusqu'à la posologie de 2 g par jour les formes pharmaceutiques les plus commodes sont les tablettes ou les capsules.

## L-METHIONINE-CAPSULES

- Formule

L-méthionine ......................... 0,50 g

Povidone (qualité pharmaceutique) .... 0,03 g

Amidon de maïs ....................... 0,02 g

- Procédé de fabrication

Granuler la L-méthionine avec une solution de 20 % de povidone dans l'éthanol. La povidone ou polyvinyl-pyrrolidone est produite industriellement sous forme d'un mélange de polymères dont le poids moléculaire moyen varie entre 10.000 et 700.000.

Si nécessaire la granulation est complétée par addition d'éthanol.

La masse humide est ensuite passée au travers d'un tamis de 3 mm. d'ouverture et le granulé humide ainsi obtenu séché à l'étuve ventilée. Le granulé sec est ensuite calibré par passage sur tamis de 1 mm.

Ajouter enfin l'amidon de maïs, mélanger et procéder à la mise en capsule selon la dose décrite précédemment.

## L-METHIONINE-COMPRIMES

### - Formule

|  |  |
|---|---|
| L-méthionine .......................... | 0,50 g |
| Phosphate tricalcique ................ | 0,25 g |
| Povidone (qualité pharmaceutique) .... | 0,05 g |
| Amidon de maïs ....................... | 0,05 g |
| Magnésium stéarate ................... | 0,02 g |
| Talc ................................. | 0,01 g |

### - Procédé de fabrication

Dans un mélangeur planétaire mélanger soigneusement la L-méthionine et le phosphate tricalcique. Humidifier ensuite le mélange par addition d'une solution à 20 % de povidone dans l'éthanol.

Compléter si nécessaire par addition d'éthanol. Granuler la masse humide par passage au travers d'un tamis de 3 mm d'ouverture. Sécher le granulé à 50°C en étuve ventilée puis calibrer le granulé sec par passage au travers d'un tamis de 1,2 mm d'ouverture.

Mélanger ensuite soigneusement le granulé avec les autres constituants de la formule (amidon de maïs, magnésium stéarate et talc).

Comprimer enfin le granulé sur machine rotative. Chaque comprimé d'un poids de 0,88 g contient 0,50 g de L-méthionine.

Quand la posologie journalière est égale ou supérieure à 5 g de L-méthionine il est préférable de prescrire le médicament sous forme de granulé à croquer.

## L-MÉTHIONINE-GRANULES

- Formule

| L-méthionine | 2,50 g |
|---|---|
| Gélatine | 0,100 g |
| Acide citrique anhydre | 0,200 g |
| Citrate trisodique | 0,100 g |
| Emkalix pluronic F.68 | 0,025 g |
| Arôme ananas 22B137 | 0,050 g |
| Aspartam | 0,025 g |
| Poids | 3,000 g |

par sachet

Adjuvant de granulation : eau purifiée

- Procédé de préparation

Dans une petite cuve en acier inoxydable de 20 l. sous agitation, faire une solution à 45-50° avec :

| Gélatine codex | 4 kg |
|---|---|
| Emkalix pluronic F.68 | 1 kg |
| Eau purifiée c.s.p. | 13,500 l. |

Charger dans la cuve du mélangeur planétaire :

| L-méthionine | 100 kg |
|---|---|
| Acide citrique | 8 kg |
| Citrate trisodique | 4 kg |

Mélanger les produits durant 15 mn et commencer le mouillage avec :

| Arôme ananas 22B137 | 2 kg |
|---|---|

puis ajouter :

| Solution de gélatine précédemment préparée | 13,5 l. |
|---|---|

Terminer éventuellement le mouillage avec :

Eau purifiée q.s.

Granuler le mélange sur granulateur type Frewitt, équipé d'une grille de 5 mm d'ouverture de maille, étaler le grain en plateaux, à raison de 3 cm d'épaisseur par plateau et sécher le grain, en étuve ventilée à 40-45° durant 36 h. minimum. Démasser le grain séché sur granulateur type Frewitt, équipé d'une grille de 3 mm d'ouverture de maille.

Dans la cuve du mélangeur planétaire, charger :

Grain démassé ......................... 119 kg env.
Aspartam ............................. 1 kg

Mélanger durant 10 mn, répartir le granulé en sachets, à raison de 3 g. par sachet.

**0088016**

Revendications de brevet

1. Médicament utile pour le traitement de syndromes dégénératifs caractérisé en ce qu'il contient en tant que principe actif de la L-méthionine.

2. Médicament utile pour le traitement de maladies associées à des désordres immunitaires caractérisé en ce qu'il contient en tant que principe actif de la L-méthionine.

3. Médicament selon la revendication 1 ou 2 sous forme de dose unitaire contenant de 2,5 g à 500 mg de substance active.

4. Médicament selon la revendication 1 utile pour le traitement du Parkinson.

5. Médicament selon la revendication 1 utile pour le traitement des troubles associés à la senescence.

6. Médicament selon la revendication 2 utile pour le traitement de l'immunodéficit.

7. Médicament selon la revendication 2 utile pour le traitement des maladies autoimmunes.

Office européen
des brevets

**RAPPORT DE RECHERCHE EUROPEENNE**

**0088016**
Numéro de la demande

EP 83 40 0383

## DOCUMENTS CONSIDERES COMME PERTINENTS

| Catégorie | Citation du document avec indication. en cas de besoin, des parties pertinentes | Revendication concernée | CLASSEMENT DE LA DEMANDE (Int. Cl. ³) |
|---|---|---|---|
| X | EP-A-0 018 550 (PLANTORGAN WERK K.G.) <br> * Revendications * <br><br> --- | 1-7 | A 61 K 31/195 |
| X | HAGERS HANDBUCH DER PHARMAZEUTISCHEN PRAXIS, vol. 3, 1972, page 18, Springer-Verlag, Berlin, DE. <br> * Page 18, "Anwendung" <br><br> --- | 1-7 | |
| X | CHEMICAL ABSTRACTS, vol. 92, no. 26, 30 juin 1980, page 326, no. 220692s, Columbus, Ohio, USA <br> & JP - A - 79 157 838 (KYOWA HAKKO KOGYO CO., LTD.) 13-12-1979 * En entier * <br><br> --- | 1-7 | |
| X | DICTIONNAIRE VIDAL, Edition 57, 1981, Paris, FR. <br> * Page 730, "Lobamine" * <br><br> --- | 1-7 | DOMAINES TECHNIQUES RECHERCHES (Int. Cl. ³) <br><br> A 61 K 31/00 |
| X | US-A-3 936 527 (DAMON CORP.) <br> * Exemple * <br><br> --- | 1-7 | |
| X | CHEMICAL ABSTRACTS, vol. 96, no. 19, 10 mai 1982, page 45, no. 155200w, Columbus, Ohio, USA <br> N.K. KHANNA et al.: "Antiinflammatory and analgesic activity of L-methionine" & ANN. NATL. ACAD. MED. SCI., 1981, 17(2), 78-89 * En entier * <br><br> ----- | 1-7 | |

Le présent rapport de recherche a ete établi pour toutes les revendications

| Lieu de la recherche | Date d achèvement de la recherche | Examinateur |
|---|---|---|
| LA HAYE | 01-06-1983 | PAUWELS G.R.A. |